(19) Europäisches Patentamt
European Patent Office
Office européen des brevets

(11) EP 0 995 424 A2

(12) **EUROPÄISCHE PATENTANMELDUNG**

(43) Veröffentlichungstag:
**26.04.2000 Patentblatt 2000/17**

(51) Int. Cl.[7]: **A61K 7/32**, A61K 7/06, A61K 7/48

(21) Anmeldenummer: **99117489.7**

(22) Anmeldetag: **10.09.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **12.09.1998 DE 19841794**

(71) Anmelder:
**Beiersdorf Aktiengesellschaft**
**20245 Hamburg (DE)**

(72) Erfinder:
- **Wolf, Florian, Dr.**
  **20251 Hamburg (DE)**
- **Schreiber, Jörg, Dr.**
  **22087 Hamburg (DE)**
- **Bünger, Joachim, Dr.**
  **64823 Gross Umstadt-Heubach (DE)**
- **Teichmann, Stefan, Dr.**
  **22607 Hamburg (DE)**
- **Traupe, Bernd**
  **22457 Hamburg (DE)**

(54) **Deodoriende Kombinationen von Antiadhäsiva (Ceramide und Sphingosine und Derivate) und Mikrobiziden**

(57) Gegenstand der Erfindung sind Zubereitungen, insbesondere kosmetische oder dermatologische, insbesondere topische Zubereitungen mit einem Gehalt an einer Kombination von

(A) einem Wirkstoff oder mehreren Wirkstoffen, ausgewählt aus der Gruppe der antiadhäsiv wirkenden Wirkstoffe (Anti-Adhäsiva),
kombiniert mit
(B) einem Wirkstoff oder mehreren Wirkstoffen, ausgewählt aus der Gruppe der antimikrobiell wirkenden Wirkstoffe oder Wirksysteme (Mikrobizide).

EP 0 995 424 A2

## Beschreibung

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Organismen sind in ihrer Eigenschaft als pathogene Keime von medizinischer Bedeutung, andere wiederum sind vollkommen unschädlich.

Gegen Bakterien wirksame Substanzen sind seit geraumer Zeit bekannt. Der Begriff „Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist das Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Wirkstoff zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch insbesondere grampositive Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosol-sprays, Roll-ons und dergleichen als auch feste Zubereitungen, beispielsweise Deo-Stifte ("Sticks"), Puder, Pudersprays, Intimreinigungsmittel usw..

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Werkstoffe und Parfümbestandteile gelegentlich miteinander reagieren und einander unwirksam machen können.

Desodorantien sollen folgende Bedingungen erfüllen:

1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsforman kosmetischer Desodorantien eingearbeitet werden können, ohne auf eine oder wenige spezielle Darreichungsformen festgelegt zu sein.

Pilze, auch Fungi [fungus = lat. Pilz], Mycota [mukhζ = grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch eine Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind kohlenstoff-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche (Onychomykosen).

Ferner sind Superinfektionen der Haut durch Pilze und Bakterien nicht selten.

Bei bestehendem Primärinfekt, d.h. der normalen Keimbesiedelung der Haut, eintretender Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüsse eine "Superinfektion" der befallenen Haut auftreten. Die normale Mikroflore der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nennenswerte Einbußen erleidet.

Protozoen sind parasitisch lebende Einzeller mit klar abgegrenztem Zellkern, die sich ungeschlechtlich fortpflanzen (durch Teilung sowie Knospung), oder aber geschlechtlich (Gameto-, Gamonto- und Autogamie). Die Nahrungsaufnahme aus der Umgebung erfolgt durch Permeation sowie durch Pino- oder Phagozytose. Die meisten Protozoen können neben vegetativen, meist beweglichen Zustandsformen (sogenannten Trophozohen) unter ungünstigen Umständen auch Zysten als Dauerformen ausbilden,

Je nach Fortbewegungsart und -apparat werden Protozoen in vier verschiedene Gruppen unterteilt:

(a) Mastigophora (Flagellaten mit Geißeln)
(b) Sarcodina/Rhizopoda (amöboides Bewegungsmuster durch Plasmaausstülpungen)
(c) Sporozoa (schlängelndes oder gleitendes Bewegungsmuster)
(d) Ciliata/Ciliophora (Bewimperung oder Begeißelung)

Parasitisch lebende Protozoen werden in subtropischen und tropischen Gebieten häufig durch stechende und saugende Insekten, aber auch Schmutz- und Schmierinfektion sowie durch die Nahrungskette übertragen.

Einige medizinisch und dermatologisch relevante Protozoonosen sind: Trichomoniasis (verursacht von Trichomonas vaginalis), Lamblienruhr (verursacht durch Lamblia intestinalis), viszerale sowie kutane und Schleimhaut-Leishmaniose (verursacht beispielsweie durch Leishmania donovanii, L.tropica, L.brasiliensis, L.mexicana, L.diffusa oder L. pifanoi), Trypanosmiasis (verursacht durch verschiedene Trypanosoma-Arten), Amöbenruhr und Amöbiasis (verursacht beispielsweise durch verschiedene EntamoebaArten, Jodamoeba butschlii oder Naegleria fowleri), Kokzidose (durch Isospora belli) und Balantidenruhr (verursacht durch Balantidium coli).

Durch Protozoonosen hervorgerufene medizinische und dermatologische Phänomene beeinträchtigen, zum Teil erheblich, das menschliche Wohlbefinden. Es besteht daher bei den betroffenen Personen ein erheblicher Bedarf, diesem Zustande abzuhelfen. Eine Aufgabe der vorliegenden Erfindung war es also, gegen Protozoen wirksame Wirkprinzipien zu finden.

Parasiten sind ein- oder mehrzellige Pflanzen oder Tiere, die sich auf (= Ektoparasiten) oder in (= Endoparasiten) anderen Lebewesen auf deren kosten ernähren, und zwar mit (= Pathogene Parasiten) oder ohne (Apathogene Parasiten) Verursachung von Krankheitserscheinungen. Die Lebensweise ist entweder auch aprophytisch oder aber rein parasitär, eventuell nur als periodischer, temporärer oder stationärer Parasit. Die Entwicklung von Parasiten ist an einen oder mehrere verschiedene Wirtsorgansimen gebunden, wobei der Mensch Zwischenwirt oder Endwirt sein

kann.

Medizinisch und dermatologisch bedeutsame Parasiten sind beispielsweise die Helminthen, die sich wiederum in Trematodae, Cestodae und Nematodae untergliedern. Das menschliche Wohlbefinden beeinträchtigende Helminthosen sind beispielsweise Bilharziose, (verursacht durch Schistosoma-Arten), Bandwurmbefall vom Darm und anderen inneren Organen (verursacht durch beispielsweise Taenia-Arten und Echinococcus-Arten), Ascariasis (verursacht durch Ascaris lumbricoides), Enterobiasis (verursacht durch Enterobium vermicularis), Paragonimiasis (verursacht durch Paragonium-Arten), Filariose (verursacht beispielsweise durch Wucheria bancrofti) sowie anderer Nematodenbefall (beispielsweise verursacht durch Trichuris trichura oder Trichinella spiralis).

Darüberhinaus bestehen eine Vielzahl auf bzw. in Mensch und Tier parasitisch lebender Insektenarten bzw. Spinnentieren, die medizinische und dermatologische Veränderungen der Wirtsorganismen hervorrufen. In dieser Hinsicht für die Beeinträchtigung des menschlichen Wohlbefindens verantwortliche Parasitosen sind beispielsweise Accrodermatitis (verursacht durch Getreidemilben, beispielsweise Pediculoides ventricosus), Skabies (verursacht durch Sarcoptes scabii), Fliegen- und/oder Fliegenlarvenbefall (verursacht beispielsweise durch Glossina-, Stomoxys-, Tabanus-, Chrysops-, Lucilia-, Chrysomya-, Cochliamya-, Wohlfartia-, Cordylobia- oder Dermatobia-Arten), Mücken- und/oder Mückenlarvenbefall (verursacht beispielsweise durch Aedes- Culex-, Anopheles-, Phlebotomus- Culicuides-, Sumilium- oder Haemagoges-Arten), Zeckenbefall (verursacht beispielsweise durch Argas persicus und andere Argas-Arten, Ornithodorus erraticus und andere Ornithodorus-Arten, Orobius-Rhiphocephalus-, Dermacentor-, Haemaphysalis-, Amblyomma-, Ixodes-Arten), Porocephalose (verursacht durch Porocephalus-Arten), Flohbefall (verursacht durch beispielsweise Pulex irritans, Ctenocephalides canis, Xenopsylla cheopsis, Nosophyllus fasciatus oder Sarcopsylla penetrans), Läusebefall (verursacht beispielsweise durch Phthirius pubis, Pediculosus humanus oder Pediculosus captits), Wanzenbefall (verursacht beispielsweise durch Cimex lectularius, Cimex hemipterus, Panstrongylus megistus, Rhodnius prolixus, Triatoma dimidata, Triatoma infestans, Triatoma sordida oder Triatoma brasiliensis) sowie Milbenbefall (verursacht beispielsweise durch Demodex folliculorum und andere Demodex-Arten sowie durch Dermamyskus-Arten Glyciphagus domesticus, Pyemotes-Arten, Sarcoptes-Arten oder Trombicula-Arten).

Dabei ist von zusätzlicher Bedeutung, daß die auf oder im menschlichen Organismus lebenden Parsiten ihrerseits wieder Überträger von Bakterien, Mycota, Protozoen und Viren sein können, die Gesundheit und Wohlbefinden des Wirtsorganismus, beispielsweise des Menschen, nachhaltig beeinträchtigen können. Es bestand daher der Bedarf, gegen Parasitosen wirksame Wirkprinzipien zu finden, welche das medizinische oder dermatologische Erscheinungsbild zu verbessern imstande sind. Diesen Bedarf zu stillen, war daher eine weitere Aufgabe der vorliegenden Erfindung.

Im Gegensatze zu den prokaryotischen und eukaryotischen zellulären Organismen sind Viren [ virus = lat. Gift ] biologische Strukturen, welche zur Biosynthese eine Wirtszelle benötigen. Extrazelluläre Viren (auch „Virionen" genannt) bestehen aus einer ein- oder doppelsträngigen Nukleinsäuresequenz (DNS oder RNS) und einem Proteinmantel (Capsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picornaviridae), Hepadnaviren (z.B. Hepatitis-B, Familie der Hepadnaviridae).

Viruzide, also Viren abtötende Substanzen im eigentlichen Sinne gibt es nicht, da Viren nicht über einen eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen sind jedenfalls schwierig. Mögliche Wirkmechanismen im Kampfe gegen die Viren sind in erster Linie die Störung deren Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie „antiviral" oder „gegen Viren wirksam", „viruzid" oder ähnlichen, die Eigenschaft einer Substanz verstanden, einen ein- oder mehrzelligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Dem Stande der Technik mangelt es jedoch an gegen Viren wirksamen Substanzen, welche zudem den Wirtsorganismus nicht oder nicht in vertretbarem Maße schädigen.

Eine Aufgabe der vorliegenden Erfindung war also, diesen Nachteilen abzuhelfen, also Substanzen zu finden, welche wirksam einen ein- oder mehrzelligen Organismus vor schädlichen Folgen einer Virusinfektion, sei es prophylaktisch oder therapeutisch, schützen.

Die erfindungsgemäßen Wirkstoffkombinationen und Zubereitungen damit können prophylaktisch verwendet werden und bewirken, daß sich nur noch geringen und keine störenden Ansammlungen von Mikroorganismen, Viren, Parasiten und Protozoen ausbilden, oder sie beseitigen auch bereits vorhandene Mikroorganismen, Viren, Parasiten und Protozoen und verringern so deren Anzahl.

Alle vorstehend und nachstehend genannten Aufgaben werden erfindungsgemäß gelöst. Die erfindungsgemäßen Wirkstoffkombinationen haben die erfindungsgemäßen, genannten Wirkungen auf Mikroorganismen, Viren, Parasiten und Protozoen und sind zur Behandlung der genannten Störungen und Krankheiten geeignet.

Mikroorganismen der Haut und die durch diese Organismen hervorgerufenen kosmetischen, dermatologischen und medizinischen Phänomene (z.B. Achselgeruch, Fußgeruch, Körpergeruch, Kopfschuppen, Akne, Superinfektionen bei atopischem Ekzem, Psoriasis oder allgemein schlechtem Immunzustand, aber auch Wundinfektionen) werden bislang im wesentlichen durch Anwendung von mehr oder weniger breitbandig wirkenden Mikrobiziden bekämpft. Die Nachteile der antimikrobiellen Therapie sind dabei die größtenteils mangelhafte Selektivität der eingesetzten Wirksysteme, Nebenreaktionen, Unverträglichkeiten und insbesondere die Ausbildung von Multiresistenzen, die eine alleinige antimikrobielle Therapie der o.a. Phänomene in naher Zukunft obsolet erscheinen lassen.

Mikroorganismen adhärieren über verschiedene Rezeptorsysteme an definierte Oberflächenstrukturen lebender und toter Zellen der menschlichen Haut. Dabei erkennen verschiedene Organismen unterschiedliche Motive und benutzen diese gezielt zur Anheftung. Es wurde erfindungsgemäß gefunden, daß im adhärierten Zustand Mikroorganismen eine deutlich geringere Sensitivität gegenüber Mikrobiziden als in Lösung bzw. Suspension zeigen, hier also eine höhere Sensitivität besitzen. Die molekularen Mechanismen, die eine derartige Sensitivitätsänderung beim Übergang vom adhärierten in den suspendierten Zustand bewirken, sind unbekannt. Es wird angenommen, daß insbesondere diese Änderung die Grundlage für den überraschenden erfindungsgemäßen Effekt bildet.

Aufgabe der Erfindung war es z.B., die genannten Nachteile des Standes der Technik zu vermeiden und mildere, hautfreundlichere Produkte zur Bekämpfung von Bakterien, Mykota, Viren, Parasiten und Protozoen zu schaffen.

Alte genannten Aufgaben wurden erfindungsgemäß gelöst und die genannten oder angestrebten Wirkungen wurden erhalten.

Gegenstand der Erfindung sind Zubereitungen, insbesondere kosmetische oder dermatologische, insbesondere topische Zubereitungen mit einem Gehalt an einer Kombination von

(A) einem Wirkstoff oder mehreren Wirkstoffen, ausgewählt aus der Gruppe der antiadhäsiv wirkenden Wirkstoffe (Anti-Adhäsiva),
kombiniert mit

(B) einem Wirkstoff oder mehreren Wirkstoffen, ausgewählt aus der Gruppe der antimikrobiell wirkenden Wirkstoffe oder Wirksysteme (Mikrobizide).

Gegenstand der Erfindung ist auch die Verwendung, insbesondere in Zubereitungen, insbesondere in kosmetischen oder dermatologischen, insbesondere topischen Zubereitungen, einer Kombination von

(A) einem Wirkstoff oder mehreren Wirkstoffen, ausgewählt aus der Gruppe der antiadhäsiv wirkenden Wirkstoffe (Anti-Adhäsiva),
kombiniert mit

(B) einem Wirkstoff oder mehreren Wirkstoffen, ausgewählt aus der Gruppe der antimikrobiell wirkenden Wirkstoffe oder Wirksysteme (Mikrobizide),

zur Bekämpfung von Bakterien, Mykote, Viren, Parasiten und Protozoen und zu der Behandlung der mit dem insbesondere kutanen Auftreten dieser Mikroorganismen verbundenen kosmetischen, dermatologischen und medizinischen Phänomene, z.B. Achselgeruch, Körpergeruch, Fußgeruch, Kopfgeruch, Akne, seborrhoische Dermatitis, mikrobielle Superinfektionen bei atopischem Ekzem, Psoriasis und Immunsuppression, sowie zur Konservierung oder bei Wundinfektionen.

Die Verwendung zur Konservierung oder als Konservierungsmittel betrifft insbesondere Kosmetika, Dermatika und Lebensmittel.

Gegenstand der Erfindung ist auch die Kombination der Wirkstoffe von (A) und (B).

Unter Ausnutzung der anti-adhäsiven Wirksamkeit verschiedener kosmetischer Rohstoffe konnte gefunden werden, daß die Anwendung von Kombinationen aus Anti-Adhäsiva und Mikrobiziden eine synergistische Steigerung der Wirksamkeit dieser Mikrobizide insbesondere gegenüber kosmetisch, dermatologisch und medizinisch relevanten Mikroorganismen bewirkt. Die Vorteile der Anwendung derartiger Wirkstoff-Kombinationen gegenüber dem Stand der Technik liegen in der bei Anwesenheit von Anti-Adhäsiva deutlich gesteigerten Wirksamkeit gleicher Konzentrationen an Mikrobizid, bzw. in der in Anwesenheit von Anti-Adhäsiva deutlich geringeren notwendigen Konzentration an Mikrobizid, um eine vergleichbare Wirkung zu erzielen. Dies ermöglicht die Entwicklung von milderen, hautfreundlicheren Produkten.

Darüber hinaus kann die Wirksamkeit der Kombination durch geschickte Auswahl des Anti-Adhäsivums maßgeschneidert werden, indem ein selektiv gegen einen bestimmten Mikroorganismus gerichtetes Anti-Adhäsivum mit einer geringen Dosis an antimikrobiellem Mittel kombiniert wird, welche *per se* gegen kutane Mikroorganismen nur wenig oder gar nicht wirksam ist. Durch die selektive Wirksamkeit des Anti-Adhäsivums wird nunmehr ein definierter Mikroorganismus von der Hautoberfläche abgelöst und damit einhergehend die Sensitivität gegenüber dem Mikrobizid deutlich erhöht. Folglich reichen bereits sehr geringe Dosen an antimikrobiellen Mittel aus, um in Anwesenheit von Anti-Adhäsiva selektiv von der Hautoberfläche abgelöste Organismen abzutöten.

Erfindungsgemäß bevorzugte Antiadhäsiva (A) sind beispielsweise die folgenden Antiadhäsiva.

$A_1$ natürliche Ceramide und Sphingosine pflanzlichen und tierischen Ursprungs, pflanzliche und tierische Rohstoffe dieselben enthaltend, sowie synthetisch hergestellte Ceramide und Sphingosine (z.B. Acyl-Ceramide, Questamide oder Pseudoceramide), aber auch durch chemische oder biologische Transformation aus natürlichen oder synthetischen Ceramiden und Sphingosinen sowie geeigneten pflanzlichen und tierischen Rohstoffen hergestellte Produkte.

Nachstehend werden einige Beispiele für die Wirksysteme angegeben:

$A_2$ Sphingosin; N-Monoalkylierte Sphingosine; N,N-Dialkylierte Sphingosine; Sphingosin-1-Phosphat; Sphingosin-1-Sulfat; Psychosin (Sphingosin-β-D-Galactopyranosid); Sphingosylphosphorylcholin; Lysosulfatide (Sphingosylgalactosylsulfat; Lysocerebrosidsulfat); Lecithin; Sphingomyelin; Sphinganin

$A_3$ humane Ceramide 1-7, humane Ceramide A und B

$A_4$ synthetische Analoga: Acyl-Ceramide, Questamide oder Pseudoceramide

$A_5$ Globotriaosylceramid; Globotetraosylceramid, Globopentaosylceramid; Forssmann-Glycolipid und Analoga

$A_6$ Glycocerebroside (e.g. Glucocerebroside, Galactocerebroside, Lactocerebroside), N-Alkanoylcerebroside (z.B. N-Palmitoylcerebrosid, N-Stearoylcerebrosid), N-Alkenoylcerebroside (e.g. N-Nervonoylcerebrosid, N-Oleoylcerebrosid), Cerebrosidsulfat

$A_7$ Ganglioside (e.g. Typen 1 bis 5, Asialogangliosid GM1, Asialogangliosid GM2, ü Asialogangliosid GM3, Monosialogangliosid GM1, Monosialogangliosid GM2, Monosialogangliosid GM3, Disialogangliosid GD1a, Disialogangliosid GD1b, Disialogangliosid GD2, Disialogangliosid GD3, Trisialogangliosid GT1b, Tetrasialogangliosid GQ1b)

$A_8$ kosmetische Rohstoffe, e.g. Ceramides GSL, Sphingoceryl LS, Sphingoceryl-Wachs LS 2958 und Sphingosomes AL (alle von Laboratoire Serobiologiques), Sphingolipid
CB-1 (Nikko), Glycocer, Glycocer-HA und -HALA (alle von Intergen), Glycoderm, Liposomes CLR und Thiosome (alle von Kurt Richter), Glycosome (Pentapharm), Phospholipids FPA und FPT (beide von Bioiberica)

Bevorzugt werden die folgenden kommerziell erhältlichen kosmetischen Rohstoffe:

| Handelsname | Lieferant |
|---|---|
| AFR LS | Serobiologiques |
| Ceramides LS | Serobiologiques |
| Dermatein GSL | Hormel |
| Lipodermol | Serobiologiques |
| Sphingoceryl LS | Serobiologiques |
| Sphingoceryl LS Powder | Serobiologiques |
| Sphingoceryl Wax LS 2958 B | Serobiologiques |
| Sphingosomes AL | Serobiologiques |

(fortgesetzt)

| Handelsname | Lieferant |
|---|---|
| Sphingolipid CB-1 | Nikko |
| GlycoCer. | Intergen |
| Glycocer.HA | Intergen |
| GlycoCer.HALA | Intergen |
| Glycoderm | Kurt Richter |
| Glycosome | Pentapharm |
| Liposomes CLR | Kurt Richter |
| Phospholipids FPA | Bioiberica |
| Phospholipids FPT | Bioiberica |
| Thiosome | Kurt Richter |

Es können z.B. auch Zweifach-, Dreifach- oder Vierfachgemische der Wirksysteme oder Wirkstoffe (A) verwendet werden.

Erfindungsgemäß geeignet sind alle Mikrobizide. Bevorzugt werden die folgenden antimikrobiellen Wirksysteme oder Wirkstoffe (B):

$B_1$ unverzweigte oder ein und mehrfach alkylverzweigte gesättigte oder ein bis fünffach ungesättigte (bis zu fünf Doppel- oder Dreifachbindungen, auch gemischte En-In-Verbindungen) Fettalkohole, -aldehyde und -säuren der Kettenlängen C2 bis C40

$B_2$ Aryl- oder Aryloxy-substituierte unverzweigte oder ein und mehrfach alkylverzweigte gesättigte oder ein bis fünffach ungesättigte (bis zu fünf Doppel- oder Dreifachbindungen, auch gemischte En-In-Verbindungen) Fettalkohole, - aldehyde und -säuren der Kettenlängen C2 bis C40

$B_3$ Mono- und Oligoglyceride (bis 4 Glycerin-Einheiten) unverzweigter oder ein und mehrfach alkylverzweigter gesättigter oder ein bis fünffach ungesättigter (bis zu fünf Doppel- oder Dreifachbindungen, auch gemischte En-In-Verbindungen) Fettalkohole (=> Mono- und Oligoglycerinmonoalkylether) und Fettsäuren (=> Mono- und Oligoglycerinmonoalkylester) der Kettenlängen C2 bis C40

$B_4$ Mono- und Oligoglyceride (bis 4 Glycerin-Einheiten) Aryl- oder Aryloxy-substituierter unverzweigter oder ein und mehrfach alkylverzweigter gesättigter oder ein bis fünffach ungesättigter (bis zu fünf Doppel- oder Dreifachbindungen, auch gemischte En-In-Verbindungen) Fettalkohole (=> Mono- und Oligoglycerinmonoalkylether) und Fettsäuren (=> Mono- und Oligoglycerinmonoalkylester) der Kettenlängen C2 bis C40

$B_5$ pflanzliche und tierische Fettsäureschnitte, enthaltend unverzweigte oder ein und mehrfach alkylverzweigte gesättigte oder ein bis fünffach ungesättigte (bis zu fünf Doppel- oder Dreifachbindungen, auch gemischte En-In-Verbindungen) Fettalkohole, -aldehyde und -säuren der Kettenlängen C2 bis C40 (z.B. Kokosfettsäuren, Palmkernfettsäuren, Wollwachssäuren)

$B_6$ unverzweigte oder ein und mehrfach alkylverzweigte gesättigte oder ein bis fünffach ungesättigte (bis zu fünf Doppel- oder Dreifachbindungen, auch gemischte En-In-Verbindungen) Alkandiole, Dialdehyde und Dicarbonsäuren der Kettenlängen C2 bis C40

$B_7$ Aryl- oder Aryloxy-substituierte unverzweigte oder ein und mehrfach alkylverzweigte gesättigte oder ein bis fünffach ungesättigte (bis zu fünf Doppel- oder Dreifachbindungen, auch gemischte En-In-Verbindungen) Alkandiole, Dialdehyde und Dicarbonsäuren der Kettenlängen C2 bis C40

$B_8$ Mono- und Oligoglyceride (bis 4 Glycerin-Einheiten) unverzweigter oder ein und mehrfachalkylverzweigter gesättigter oder ein bis fünffach ungesättigter (bis zu fünf Doppel- oder Dreifachbindungen, auch gemischte En-In-Verbindungen) Alkandiole (=> Mono- und Oligoglycerinmonoalkylether; Bis(mono-/oligoglyceryl)alkyldiether) und Dicarbonsäuren (=> Mono- und Oligoglycerinmonoalkylester; Bis(mono- /oligoglyceryl)alkyldiester)

der Kettenlängen C2 bis C40

$B_9$ Mono- und Oligoglyceride (bis 4 Glycerin-Einheiten) Aryl- oder Aryloxy-substituierter unverzweigter oder ein und mehrfach alkylverzweigter gesättigter oder ein bis fünffach ungesättigter (bis zu fünf Doppel- oder Dreifachbindungen, auch gemischte En-In-Verbindungen) Alkandiole (=> Mono- und Oligoglycerinmonoalkytether; Bis(mono-/oligoglyceryl)alkyldiether) und Dicarbonsäuren (=> Mono- und Oligoglycerinmonoalkylester; Bis(mono-/oligoglyceryl)-alkyldiester) der Kettenlängen C2 bis C40

$B_{10}$ pflanzliche und tierische Fettsäureschnitte, enthaltend unverzweigte oder ein und mehrfach alkylverzweigte gesättigte oder ein bis fünffach ungesättigte (bis zu fünf Doppel- oder Dreifachbindungen, auch gemischte En-In-Verbindungen) Alkandiole, Dialdehyde und Dicarbonsäuren der Kettenlängen C2 bis C40

$B_{11}$ Fettsäureester unverzweigter oder ein und mehrfach alkylverzweigter gesättigter oder ein bis fünffach ungesättigter (bis zu fünf Doppel- oder Dreifachbindungen, auch gemischte En-In-Verbindungen). ggf. auch Aryl- oder Aryloxy-substituierter Carbonsäuren der Kettenlängen C2 bis C40 mit unverzweigten oder ein und mehrfach alkylverzweigten gesättigten oder ein bis fünffach ungesättigten (bis zu fünf Doppel- oder Dreifachbindungen, auch gemischte En-In-Verbindungen), ggf. auch Aryl- oder Aryloxy-substituierten ein- bis sechswertigen Fettalkoholen der Kettenlängen C2 bis C40

$B_{12}$ Ein- und mehrfach halogenierte Nitrile, Dinitrile, Trinitrile oder Tetranitrile der Struktur

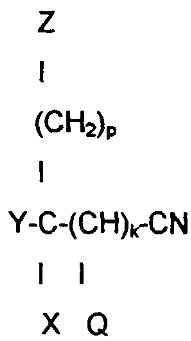

wobei Q ein Halogen- oder Wasserstoffatom darstellt und wobei X, Y und Z unabhängig voneinander gewählt werden aus der Gruppe Q, -CN, -H, verzweigter oder unverzweigter Methyl- bis Octadecyl-Rest, p = 0 - 10 und k = 0 - 18.

$B_{13}$ Phenylverbindungen der Struktur

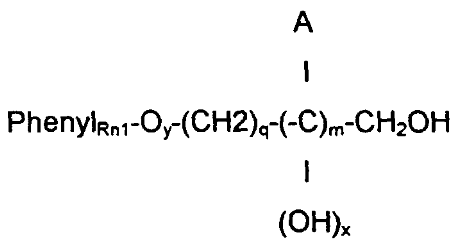

mit $R_1$ = H, Methyl, Methoxy oder Amino, und wobei bis zu fünf gleiche oder verschiedene Reste $R_1$ bzw. beliebige Kombinationen gleicher und verschiedener solcher Reste innerhalb eines Moleküls auftreten können, entsprechend n = 1 bis 5, wobei A darstellen kann: H, Methyl, Ethyl, mit q = 1 bis 10, m = 1 bis 10, y = 0 oder 1 und x = 0 oder 1, und wobei innerhalb eines Moleküls, wenn m > 1, für einzelne Kohlenstoffatome x identische, aber auch unterschiedliche Werte annehmen kann.

$B_{14}$ Mono- und Oligohydroxyfettsäuren der Kettenlängen $C_2$ bis $C_{24}$ (z.B. Milchsäure, 2-Hydroxypalmitinsäure), deren Oligo- und/oder Polymere sowie pflanzliche und tierische Rohstoffe, dieselben enthaltend

$B_{15}$ unsubstituierte und Alkylsubstituierte Hydrochinone sowie Pflanzenextrakte, dieselben enthaltend (z.B. Salbeiextrakt, Rosmarinextrakt)

$B_{16}$ Terpene

Acyclische Terpene:Terpenkohlenwasserstoffe (z.B. Ocimen, Myrcen), Terpenalkohole (z.B. Geraniol, Linalool, Citronellol), Terpenaldehyde und -ketone (z.B. Citral, Pseudoionon, β-Ionon); Monozyklische Terpene: Terpenkohlenwasserstoffe (z.B. Terpinen, Terpinolen Limonen), Terpenalkohole (z.B. Terpineol, Thymol, Methol), Terpenketone (z.B. Pulegon, Carvon) Bizyklische Terpene: Terpenkohlenwasserstoffe (z.B. Caran, Pinan, Bornan), Terpenalkohole (z.B. Borneol, Isoborneol), Terpenketone (z.B. Campher), Sesquiterpene: Acyclische Sesquiterpene (z.B. Farnesol, Nerolidol), Monocyclische Sesquiterpene (z.B. Bisabolol), Bicyclische Sesquiterpene (z.B. Cadinen, Selinen Vetivazulen, Guajazulen), Tricyclische Sesquiterpene (z.B. Santalen), Diterpene (z.B. Phytol), Tricyclische Diterpene (z.B. Abietinsäure), Triterpene (Squalenoide; z.B. Squalen), Tetraterpene

$B_{17}$ Halogenierte aromatische Verbindungen (z.B. Trichlorocarbanilid, Hexachlorophen, Triclosan, Dichlorphenylether etc.)

$B_{18}$ klassische Konservierungsmittel (z.B. Formaldehyd, Glutardialdehyd, Mezetroniumethylsulfat, Parabene (z.B. Methyl-, Ethyl-, Propyl- und Butylparaben), Sorbit, Dibromdicyanobutan, Imidazolidinylharnstoffe („Germall"), Diazohidinylharnstoff, Isothiazolinone („Kathon"), Methylchlorthiazolidin, Methylthiazolidin, organische Säuren (z.B. Benzoesäure, Sorbinsäure, Salicylsäure) sowie deren Ester, Glycole (z.B. Propylenglycol, 1,2-Dihydroxyalkane), pflanzliche Konservierungshelfer und Flavonoide (z.B. Lantadin A, Caryophyllen, Hesperidin, Diosmin, Phellandren, Apigenin, Quercetin, Hypericin, Aucubin, Diosgenin, Plumbagin, Corlilagin etc.) sowie deren glycosylierte Derivate (z.B. Glycosylrutin)

$B_{19}$ Ethoxylierte, propoxylierte oder gemischt ethoxylierte/propoxylierte kosmetische Fettalkohole, Fettsäuren und Fettsäureester der Kettenlängen C2 bis C40 mit 1 bis 150 E/Q- und/oder P/O-Einheiten

$B_{20}$ antimikrobielle Peptide und Proteine mit einer Aminosäurezahl von 4 bis 200, z.B. Magainine, Magainin-Amide, PGLa, PYLa, PGSa, Xenopsin, Xenopsin Precursor Fragments [XPFs], Caerulein, Caerulein Precursor Fragments [CPFs], Caeridine, Brevinine, Esculentine, Bombinine, Dermaseptine, Tachyplesine, Polyphemusine, Lantibiotika [z.B. Epidermin, Gallidermin, Nisin, Subtilin, Pep5, Pediocine, Plantaricine, Leucocine, Cinnamycin, Duramycin, Ancovenin, Colicine, Pyocine, Bacteriocine, Microcine, Lactococcine, Lactacine, Mersacidine, Actagardine, Lacticine, Streptococcine, Salivarine, Carnocine, Lactocine, Lanthiopeptine etc.], Skin Antimicrobial Peptides (SAPs), Lingual Antimicrobial Peptides (LAPs), humane β-Defensine (insbes. h-BD1 und h-BD2), Tracheal Antimicrobial Peptide (TAPs),Defensine, Neutrophil-Peptide [z.B. NP-1 bis NP-5; HNP-1 bis HNP-4; GPNP; Cryptidine; RatNP-1 bis RatNP-4, Sapecine, Drosocine, Cecropine, Andropine, Attacine, Sarcotoxine, Diptericine, Coelopterine, Apidaecine, Abaecine, Hymenoptaecine, Melittine, *Aedes aegyptii*-Defensine, Cathepsin D, Azurocidine, Lactoferrine und deren Hydrolysate sowie daraus gewonnene Peptide, Bactericidal/Permeability Increasing Proteins [BPIs], Elastasen, Cationic Microbial Proteins [CAPs], Lysozym, Serprocidine, Myeloperoxidase, Ondolicidine; Major Basic Proteins [MBPs], Eosinophil Cationic Proteins [ECPs]; Bactenecine; Macrophage Cationic Peptides [MCPs], Histatine, Amoebapore, Thionine, Cysteinreiche antimikrobielle Peptide aus Pflanzen (z.B. Mj-AMPs, Ac-AMPs, Rs-AFPs, Rs- nsLTPs, Rs-2S) ud deren synthetische Analoga enthaltend L-und/oder D-Aminosäuren (z.B. MSI-78)

$B_{21}$ Kohlenhydrate und Kohlenhydrat-Derivate

Gut geeignete Kohlenhydrate oder „Kohlenhydrat-Derivate", die sprachlich kurzgefaßt auch unter die Bezeichnung „Kohlenhydrate" fallen sollen, sind Zucker und substituierte Zucker oder Zuckerreste enthaltende Verbindungen. Zu den Zuckern zählen insbesondere auch jeweils die Desoxy- und Didesoxy-Formen.

Nachstehend werden einige Beispiele für die Wirksysteme angegeben:

$B_{22}$ Monosaccharide

Gut geeignete Monosaccharide sind z.B. Tetrosen, Pentosen, Hexosen und Heptosen. Bevorzugt werden Pentosen und Hexosen. Die Ringstrukturen umfassen Furanosen und Pyranosen, umfaßt sind sowohl D- als auch

L-Isomere, ebenso wie α- und β-Anomere. Geeignet sind auch die Desoxy- und Didesoxy-Formen.

$B_{23}$ Disaccharide

Gut geeignete Disaccharide sind z.B. die durch binäre Verknüpfungen obiger Monosaccharide gebildeten Disaccharide. Verknüpfung kann als α- oder β-glycosidische Bindung zwischen den beiden Untereinheiten erfolgen. Saccharose, Maltose, actobiose werden bevorzugt. Ebenso geeignet sind N-Acetyl-Galactosamin- und N-Acetyl- Glucosamin-Derivate sowie Silalinsäure-substituierte Derivate.

$B_{24}$ Oligosaccharide (natürlichen und synthetischen Ursprungs)

Gut geeignete Oligosaccharide bestehen aus mehreren, z.B. 2 - 7 Zuckereinheiten, vorzugsweise der unter Mono- und Disaccharide beschriebenen Zucker, insbesondere aus 2 bis 5 Einheiten in den bekannten, durch Kondensation entstandenen Bindungsformen und wie vorstehend genannt. Besonders bevorzugte Oligosaccharide sind neben den Disacchariden die Tri- und Tetrasaccharide. Ebenso geeignet sind N-Acetyl-Galactosamin- und N-Acetyl-Glucosamin-Derivate sowie Silalinsäure-substituierte Derivate.

$B_{25}$ Aminozucker

Gut geeignet sind Mono-, Di- und Oligosaccharide, insbesondere wie vorstehend beschrieben, mit einer oder mehreren Aminogruppen, die acyliert, insbesondere acetyliert sein können. Bevorzugt werden Ribosylamin; N-Acetylglucosamin und N-Galactosylamin sowie Sialinsäure-substituierte Derivate.

$B_{26}$ Zuckerester

Weiterhin werden Zuckerester von organischen oder anorganischen Säuren vorteilhaft verwendet, beispielsweise Zuckerphosphate, Zuckerester mit Carbonsäuren oder sulfatierte Zucker, insbesondere Ester der vorstehend beschriebenen Zucker.

$B_{27}$ Zuckerester anorganischer Säuren

Bevorzugte Zuckerester der Phosphorsäure sind Glucose-1-phosphat; Fructose-1-phosphat, Glucose-6-phosphat oder Mannose-6-phosphat.

$B_{28}$ Zuckerester organischer Säuren

Bevorzugte Ester aus Zuckern und Carbonsäuren werden mit Carbonsäuren der Kettenlänge $C_1$ bis $C_{24}$, z.B. erhalten, zum Beispiel Cetearylglucosid (Fa. Seppic: Montanol 68); Caprylyl/Caprylglucosid (Fa. Seppic: Oramix CG-110); Decylglucosid (Fa. Seppic: Oramix NS-10), Sucroselaurat und -myristat (Fa. Ryoto Sugar), Sucrose Cocoat (Fa. Croda) insbesondere aber auch die Zuckeracetate, bevorzugt der vorstehenden Zucker.

$B_{29}$ Zuckerether

Bevorzugt werden auch die Zuckerether aus Zuckern, insbesondere der vorstehenden Zucker, mit ein- und mehrwertigen Alkoholen der Kettenlänge $C_1$ bis $C_{24}$, z.B. Plantaren$^R$ 1200 (Fa. Henkel) oder Plantaren$^R$ 2000 (Fa. Henkel) (auch bekannt als Plantacare 1200 und Plantacare 2000)).

Weiterhin sind z.B. die Umsetzungsprodukte von Zuckern mit Ethylenoxid und/oder Propylenoxid geeignet, vorzugsweise mit den vorstehenden Zuckern. Geeignet sind E/O- bzw. P/O-Grade von einer bis 40 Ethereinheiten.

$B_{30}$ Glykolipide (natürlichen und synthetischen Ursrungs)

Bevorzugte Glykolipide sind neben Glycosylglycerolen, Monoacylglycosylglycerolen und Diacylglycosylglycerolen Glykosphingolipide, insbesondere Ceramide, Cerebroside, Ganglioside und Sulfatide (z.B. Globotriaosylceramid; Globotetraosyceramid, Globopentaosylceramid; Forssmann-Glycolipid und Analoga); Glycocerebroside (e.g. Glucocerebroside, Galactocerebroside, Lactocerebroside), N-Alkanoylcerebroside (z.B. N-Palmitoylcerebrosid, N-Stearoylcerebrosid), N-Alkenoylcerebroside (e.g. N-Nervonoylcerebrosid, N-Oleoylcerebrosid), Cerebrosidsulfat sowie Ganglioside (e.g. Typen 1 bis 5, Asialogangliosid GM1, Asialogangliosid GM2, Asialogangliosid GM3, Monosialogangliosid GM1, Monosialogangliosid GM2, Monosialogangliosid GM3, Disialogangliosid GD1a, Disialogangliosid GD1b, Disialogangliosid GD2, Disialogangliosid GD3, Trisialogangliosid GT1b, Tetrasialogangliosid GQ1b) sowie Tenside auf Chitinbasis

$B_{31}$ Polysaccharide (natürlichen und synthetischen Ursprunges)

Die Polysaccharide können unverzweigt oder verzweigt sein und es sind sowohl die Homopolysaccharide als auch die Hetero-Polysaccharide, jeweils insbesondere mit solchen Zuckern, wie oben beschrieben, geeignet. Bevorzugte Polysaccharide sind Stärke, Glykogen, Cellulose, Dextran, Tunicin, Inulin, Chitin, insbesondere Chitosane, Chitinhydrolysate, Alginsäure und Alginate, Pflanzengumme, Körperschleime, Pektine, Mannane,

Galactane, Xylane, Araban, Polyosen, Chondroitinsulfate, Heparin, Hyaluronsäure und Glycosaminoglykane, Hemicellulosen, substituierte Cellulose und substituierte Stärke, insbesondere jeweils die hydroxyalkylsubstituierten Polysaccharide.
Besonders geeignet sind Amylose Amylopektin, Xanthan, α-, β- und γ-Dextrin.
Die Polysaccharide können z.B. aus 4 bis 1 000 000, insbesondere 10 bis 100 000 Monosacchariden bestehen. Vorzugsweise werden jeweils solche Kettenlängen gewählt, die gewährleisten, daß der Wirkstoff in der jeweiligen Zubereitung löslich oder in sie einzuarbeiten ist.
Die erfindungsgemäßen Wirkstoffe können einzeln eingesetzt werden. Es ist aber auch möglich, zwei, drei oder auch mehrere Wirkstoffe zusammen zu verwenden.
Insbesondere können Monosaccharide und Oligosaccharide kombiniert werden, wobei jeweils ein Saccharid aber auch zwei oder drei oder mehrere Zucker gewählt werden können. Zusammen mit den vorstehend genannten Zuckern oder deren Kombinationen können vorteilhaft ein Polysaccharid oder auch mehrere Polysaccharide verwendet werden.
Fucose, Galactose, Sialinsäure, N-Acetyl-Galactosamin und N-Acetyl-Glucosamin werden besonders bevorzugt.
Bevorzugt werden die folgenden Kombinationen und Zubereitungen damit und deren Verwendungen.
Bevorzugt werden Wirkstoffkombinationen mit mindestens drei Wirkstoffen, ausgewählt aus der Gruppe enthaltend:

- Aldopentosen und Ketopentosen und
- Aldohexosen und Ketohexosen und
- Aldoheptosen und Ketoheptosen.

Die genannten Zucker können insbesondere auch in ihrer Desoxy- und Didesoxy-Form und insbesondere auch in der Form der erfindungsgemäßen Derivate vorliegen.
Dies gilt auch für die folgenden bevorzugten Kombinationen.
Besonders bevorzugt werden Kombinationen, insbesondere Kombinationen von mindestens drei Wirkstoffen, die mindestens einen Desoxy- oder Didesoxy-Zucker oder mindestens ein Desoxy- oder Didesoxy-Zucker-Derivat oder mindestens ein Disaccharid oder mindestens ein Trisaccarid oder mindestens ein Tetrasaccharid enthalten, wobei diese auch jeweils in der Form der erfindungsgemäßen Derivate oder auch in der Desoxy- oder Didesoxy-Form vorliegen können. Weiterhin werden Kombinationen, insbesondere Kombinationen von mindestens drei Wirkstoffen bevorzugt, die Fucose, Galactose, Sialinsäure, N-Acetyl-Galactosamin und N-Acetyl-Glucosamin enthalten, wobei diese auch jeweils in der Form der erfindungsgemäßen Derivate vorliegen können.
Besonders bevorzugt werden die folgenden Wirkstoffkombinationen a) - f):

a) Fucose, Raffinose und Galactose
b) Glucose-6-phosphat, Mannose-6-phosphat und Mannose
c) Raffinose, N-Acetyl-glucosamin, und Fucose
d) Mannose, Rhamnose und Fucose
e) Galactose, N-Acetyl-glucosamin und Fucose
f) Mannose, Raffinose und Galactose.

Bevorzugt werden auch die jeweiligen Einzelkomponenten der Kombinationen und die mit jeweils zwei Komponenten zu bildenden Zweier-Kombinationen aus den drei Wirkstoffen jeweils einer Dreier-Kombination.
Vorteilhaft können auch jeweils mit einem Zucker oder mehreren Zuckern aus der Gruppe der Monosaccharide und/oder der Oligosaccharide ein oder mehrere Zucker aus der Gruppe der Zuckerphosphate und/oder der Aminozucker und Acetylaminozucker kombiniert werden.
In den Unterkombinationen der Kohlenhydrate können die Wirkstoffe z.B. mit gleichen Gewichtsmengen oder auch z.B. im Gewichtsverhältnis von 1 : 1000 bis 1000 : 1, vorzugsweise 1 : 10 bis 10 : 1 verwendet werden, jeweils bezogen auf eine andere Komponente oder mehrere andere Komponenten.

$B_{32}$ natürliche Sterole und Sterol-Derivate pflanzlichen, pilzlichen und tierischen Ursprungs, pflanzliche, pilzliche und tierische Rohstoffe, dieselben oder Vorstufen dazu enthaltend, synthetische Sterole und Sterol-Derivate sowie semi-synthetisch entweder durch biologische oder chemische Transformation natürlicher und synthetischer Substanzen erhaltene Sterole und Sterol-Derivate.

Nachstehend werden einige Beispiele für die beanspruchten Sterole und Sterol-Derivate angegeben (ohne

Anspruch auf Vollständigkeit):

$B_{33}$ Gonan, Cholestan, Cholestanol, Ergosterol (aus Hefen und Pilzen), Stigmasterol (aus Sojabohnen), Gallensäuren (z.B. Cholansäure, Cholsäure, Desoxycholsäure, Chenodesoxycholsäure oder Lithocholsäure) sowie deren Salze

$B_{34}$ Cholesterol; Cholesterylether resp. -ester gesättigter sowie ein und mehrfach (bis n = 4) unverzweigte oder ein und mehrfach alkylverzweigter, gesättigter oder ein bis vierfach ungesättigter (bis zu vier Doppel- oder Dreifachbindungen, auch gemischte En-In-Verbindungen) Fettalkohole resp. Fettsäuren der Kettenlängen C2 bis C40 (z.B. Cholesterylhydroxystearat, Fa. Nissin Oil Mills; Cholesterylisostearat, Fa. Kao) ; ethoxylierte und propoxylierte Cholesterole (z.B. Choleth-20, Fa. Nikko; Choleth-24, Fa. Fanning); Dihydrocholesterol und seine Derivate (z.B. Dihydrochrolesteryloctadecanoat, Dihydrocholeth-20 und -30, alle Fa. Nikko)

$B_{35}$ Phytosterole (z.B. Soya Sterol) und Phytosteryl-Derivate (z.B. PEG-5-Soya Sterol, PEG-40-Soya Sterol, PEG-25-Phytosterol, Soya Sterol Esters) sowie Dihydrophytosterol und -Derivate (z.B. Dihydrophytosteryloctadecanoat, Fa. Nikko)

$B_{36}$ Lanosterol (Fa. Croda oder Fa. Nikko), Sitosterol, Koprosterol und deren Derivate (z.B. β-Sitosterylacetat, Fa. Variati; Dinatrium Sitostereth-14 Sulfosuccinat, Fa. Rewo)

$B_{37}$ Lanolin, Lanolinalkohol (z.B. Eucerit, Fa. Beiersdorf) und Lanolinsäure (z.B. Fa. Croda) sowie deren ethoxylierte und/oder propoxylierte Derivate (z.B. Laneth-5, Laneth-50, Laneth-10-acetat, alle Fa. Croda; PEG-75-Lanolin, Fa. Westbrook; PEG-100-Lanolin, Fa. Croda; PPG-5-Lanolin Wax, Fa. Henkel; PPG-12-PEG-50-Lanolin, PPG-12-PEG-65-Lanolin Oil, alle Fa. Croda; PPG-2-Lanolin Alkohol Ether, PPG-10-Lanolin Alkohol Ether und PPG-30-Lanolin Alkohol Ether, alle Fa. Amerchol); Mono- und Oligoglyceride des Lanolins, von Lanolinalkoholen und Lanolinsäuren (z.B. Glyceryl Lanolat, Fa. Brooks; Neocerit, Fa. Beiersdorf)

$B_{38}$ Sterole aus Tallöl sowie deren ethoxylierte und/oder propoxylierte Derivate (z.B. PEG-5-Tallöl Sterol Ether)

$B_{39}$ Glycyrrhezitinsäure (Fa. Ichimaru Pharmacos) und Derivate (z.B. Glycyrrhetinyl Stearate; Fa. Maruzen oder Fa. Ichimaru Pharmacos)

$B_{40}$

$B_{41}$ Steroidhormone natürlichen und synthetischen Ursprungs (z.B. Androgene, Östrogene, Gestagene, Corticoide, Mineralocorticoide oder Ecdyson)

$B_{42}$ natürliche und synthetische Cardenolide (z.B. Digitoxin, Dogoxin, Digoxygenin, Gitoxygenin, Strophanthin und Strophanthidin)

$B_{43}$ natürliche und synthetische Bufadienolide (z.B. Scillaren A, Scillarenin und Bufotalin)

$B_{44}$ Sapogenine und Steroid-Sapogenine (z.B. Amyrine, Oleanolsäure, Digitonin, Gitogenin, Tigogenin und Diosgenin)

$B_{45}$ Steroid-Alkaloide pflanzlichen und tierischen Ursprungs (.z.B. Tomatidin, Solanin, Solanidin, Conessin, Batrachotoxin und Homobatrachotoxin)

Bevorzugt werden die nachstehenden kommerziell erhältlichen Rohstoffe:

| Substanz | Handelsname | Lieferant |
|---|---|---|
| Beta-Sitosterol | | |
| Beta-Sitosterylacetat | Sitostearyl Complex | Variati |
| Cholecalciferol | Epiderm-Complex O | |
| Cosmetochem | | |
| Cholesterol | Cholesterol USP/NF | Croda |
| Cholesteryl Hydroxystearat | Estemol CHS | beide |
| Nissin | | |
| | Salacos HS | Oil Mills |
| Cholesteryl Isostearat | IS-CE | Kao |
| Corp. | | |
| Choleth-20 | Nikkol Aquasome EC-5 | Nikko |
| Choleth-24 | Fancol CH-24 | Fanning |
| Dihydrocholesterol | | |
| Dihydrocholesteryl Octyldecanoat | Nikkol DCIS | Nikko |
| Dihydrocholeth-15 | Nikkol DHC-15 | Nikko |
| Dihydrocholeth-20 | Nikkol DHC-20 | Nikko |
| Dihydrocholeth-30 | Nikkol DHC-30 | Nikko |

| Substanz | Handelsname | Lieferant |
|---|---|---|
| Dihydrolanosterol | Isocholesterol EX | Nikko |
| Dihydrophytosteryl Octyldecanoat | Nikkol DPIS | Nikko |
| Disodium Sitostereth-14 Sulfosuccinat Chem. | Rewoderm SPS | Rewo |
| Ergocalciferol | | |
| Glyceryl Lanolat | Ivarlan 3360<br>Neocerit | Brooks<br>BDF |
| Glycyrrhecitinsäure | 18ß-Glycyrrhecitinic acid | Ichimaru Pharmacos |
| Glycyrrhetinyl Stearat | Grhetinol-O<br>SGS | beide Maruzen |
| Laneth-5 | Polychol-5 | Croda |
| Laneth-10 | Polychol-10 | Croda |
| Laneth-15 | Polychol-15 | Croda |
| Laneth-20 | Polychol-20 | Croda |
| Laneth-25 | Soluan 25 | Amerchol |
| Laneth-50 | Sterol LN 50 | Auschem |
| Laneth-10 Acetat | Lipolan 98 | Lipo |

| Substanz | Handelsname | Lieferant |
|---|---|---|
| Lanosterol | Lanosterol | Croda |
| | Isocholesterol EX | Nikko |
| PEG-5-Lanolat | Agnosol 5 | Croda |
| PEG-10-Lanolat | Sklirate 10 | Croda |
| PEG-20-Lanolat | Sklirate 20 | Croda |
| PEG-10-Lanolin | Nikkol TW-10 | Nikko |
| PEG-20-Lanolin | Solan 20 | Croda |
| PEG-30-Lanolin | Nikkol TW-30 | Nikko |
| PEG-40-Lanolin | Solan 40 | Croda |
| PEG-75-Lanolin | Aqualose L75 | Westbrook L. |
| PEG-100-Lanolin | Aqualose L100 | Westbrook L. |
| PEG-150-Lanolin | Solan X | Croda |
| PEG-25-Phytosterol | | |
| PEG-5-Soya Sterol | | |
| PEG-10-Soya Sterol | | |
| PEG-25-Soya Sterol | | |

| Substanz | Handelsname | Lieferant |
|---|---|---|
| PEG-40-Soya Sterol | | |
| PEG-5 Tall Oil Sterol Ether | | |
| PPG-2 Lanolin Alcohol Ether | Soluan PB-2 | Amerchol |
| PPG-5-Lanolin Alcohol Ether | Soluan PB-5 | Amerchol |
| PPG-10-Lanolin Alcohol Ether | Soluan PB-10 | Amerchol |
| PPG-20-Lanolin Alcohol Ether | Soluan PB-20 | Amerchol |
| PPG-30-Lanolin Alcohol Ether | Soluan PB-30 | Amerchol |
| PPG-5-Lanolin Wax | Propoxyol 1695 | Henkel |
| PPG-12-PEG-50 Lanolin | Lanexol AWS | Croda |
| PPG-12-PEG-65 Lanolin Oil | Fluilan AWS | Croda |
| PPG-40-PEG-60 Lanolin Oil | Aqualose LL 100 | Westbrook L. |
| Stearyl Glycyrrhetinate | Co-Grhetinol | Maruzen |
| | ST-Glycyrrhetinate | Ichimaru Ph. |
| Soy Sterol | | |
| Soy Sterol Acetate | | |
| Tall Oil Sterol | | |
| C10 -C30 Cholesterol/Lanosterol Ester | | |

Bevorzugte Antiadhäsiva (A) sind die Stoffe der Stoffgruppen $A_2$, $A_5$, $A_7$ und $A_8$.Besonders bevorzugte Antiadhäsiva sind die Stoffe der Stoffgruppen $A_7$ und $A_8$, insbesondere die dort genannten Einzelverbindungen.

Bevorzugte antimikrobielle Wirksysteme oder Wirkstoffe (B) sind die Stoffe der Stoffgruppen $B_1$, $B_2$, $B_3$, $B_4$,

$B_6$, $B_7$, $B_8$, $B_9$, $B_{10}$ und $B_{11}$. Besonders bevorzugte antimikrobielle Wirkstoffe (B) sind die Stoffe der Stoffgruppen $B_3$, $B_4$, $B_8$ und $B_9$, insbesondere die dort genannten Einzelverbindungen.

Bevorzugt werden erfindungsgemäße Kombinationen von (A) und (B), die von Stoffen der bevorzugten Antiadhäsiva (A) und bevorzugten antimikrobiellen Wirkstoffen (B) gebildet werden, insbesondere von den dort jeweils genannten Einzelverbindungen und jeweils die Zubereitungen damit.
Auch die Verwendung der vorstehenden Stoffe und Kombinationen wird bevorzugt.

Bevorzugt werden die in den Beispielen genannten Wirkstoffe (A) oder (B). Bevorzugt werden auch Kombinationen der Wirkstoffe (A) und (B), die in den Beispielen genannt sind. Besonders bevorzugt werden Kombinationen der jeweils in einem Beispiel genannten Wirkstoffe (A) und (B),

Vorzugsweise beträgt die Menge der erfindungsgemäßen Antiadhäsiva (A) in den Zubereitungen, insbesondere topischen Zubereitungen, 0,2 bis 20 Gew.-%, bezogen auf das Gesamtgewicht der Zubereitungen.

Vorzugsweise beträgt die Menge der erfindungsgemäßen Mikrobizide (B) in den Zubereitungen, insbesondere topischen Zubereitungen, 0,05 bis 5 Gew.-%, bezogen auf das Gesamtgewicht der Zubereitungen.

Vorzugsweise ist die Gewichtsmenge der Antiadhäsiva (A) gleich der Gewichtsmenge oder größer als die Gewichtsmenge der Mikrobizide (B), und sie kann insbesondere bis zum Zehnfachen der Mikrobizidmenge betragen.

Besonders bevorzugt werden Gewichtsmengen der Antiadhäsiva (A), die bis zum Fünffachen der Gewichtsmenge von (B) betragen, insbesondere aber z.B. das Zweifache, Dreifache oder Vierfache betragen.

Auch die Verwendung der vorstehenden Stoffe und Kombinationen wird bevorzugt.

Die Aufbereitung der erfindungsgemäßen Wirkstoffe erfolgt nach den dem Fachmann geläufigen, üblichen Methoden. Erfindungsgemäße Wirkstoffe sind im Handel erhältlich oder bekannt oder können nach bekannten Verfahren erhalten werden.

Die Wirkstoffkombinationen sind gegenüber, insbesondere den vorstehend genannten Mikroorganismen, Viren, Parasiten und Protozoen hervorragend wirksam und zur Behandlung der genannten Krankheiten und Zustände geeignet.

Die erfindungsgemäßen Wirkstoffkombinationen wirken insbesondere im Sinne von desinfizierenden Wirkstoffen, z.B. einer die Mikroorganismen, Viren, Parasiten und Protozoen abtötenden Wirkung oder z.B. in einer Wachstumshemmung für diese. Diese Wirkprinzipien werden insbesondere auch mit den Begriffen antimikrobielle, antivirale, antiparasitäre und gegenüber Protozoen antiparasitäre Wirkung gegenüber Mikroorganismen, Viren, Parasiten und Protozoen bezeichnet und sollen in gleicher Weise auch für diese Wirkstoffkombinationen gelten.

Es hat sich in erstaunlicher Weise herausgestellt, daß die erfindungsgemäßen Wirkstoffkombinationen das Wachstum von grampositiven und gramnegativen Bakterien, Mycobionten, Protozoen, Parasiten sowie Viren verhindern. Dabei wirken die erfindungsgemäßen Wirkstoffe in synergistischer Weise, also überadditiv in bezug auf die Einzelkomponenten (A) oder (B).

Insbesondere sind die erfindungsgemäßen Wirkstoffkombinationen befähigt, das Wachstum von Hefen, insbesondere der Pityrosporum-Arten, namentlich Pityrosporum ovale, zu verhindern.

Es hat sich ferner herausgestellt, daß die erfindungsgemäßen Wirkstoffkombinationen die Bildung von seborrhoischen Erscheinungen, insbesondere Kopfschuppen, verhindern sowie bereits vorhandene seborrhoische Erscheinungen, insbesondere Kopfschuppen, beseitigen.

Die erfindungsgemäßen Wirkstoffkombinationen eignen sich darüberhinaus gut für die Verwendung als desodorierender Wirkstoff in kosmetischen Desodorantien sowie gegen unreine Haut, leichte Formen der Akne bzw. Propionibakterium acnes.

Schließlich hat sich herausgestellt, daß die erfindungsgemäßen Wirkstoffkombinationen den Verderb organischer Substanz, insbesondere kosmetischer und dermatologischer Zubereitungen, durch den Befall mit grampositiven und gramnegativen Bakterien, Mycobionten, Protozoen, Parasiten und Viren verhindern können, wenn sie diesen Zubereitungen zugesetzt werden.

Erfindungsgemäß sind somit auch ein Verfahren zur Bekämpfung von Mycobionten, dadurch gekennzeichnet, daß die erfindungsgemäßen Wirkstoffkombinationen gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, daß diesen organischen Produkten die erfindungsgemäßen Wirkstoffkombinationen in wirksamer Menge zugegeben werden.

Der Stand der Technik lieferte folglich nicht den geringsten Hinweis auf die erfindungsgemäße Verwendung als antimycotisches Wirkprinzip.

Ferner war erstaunlich, daß die erfindungsgemäßen Wirkstoffkombinationen besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Anti-Schuppen-Schampoos.

Die Anwendung der Wirkstoffkombinationen kann topisch, perkutan, transdermal, parenteral, oral oder auch intravasal erfolgen.

Zubereitungen, die erfindungsgemäße Wirkstoffkombinationen enthalten, können topische Zubereitungen sein, beispielsweise kosmetische und dermatologische topische Zubereitungen oder aber auch übliche Arzneimittel-Darreichungsformen. Bevorzugt werden Desodorantien oder desodorierende Körperreinigungsprodukte oder Körperpflegeprodukte. Die Wirkstoffkombinationen können aber auch in Desinfektionsmitteln und/oder Reinigungsmitteln enthalten sein, die nicht nur zur behandlung des Körpers oder der Haut bestimmt sind, sondern auch zum Reinigen und Desinfizieren von harten Oberflächen, medizinischen Materialien, Geräten, Instrumenten, Mobiliar und Wänden.

Für den Körper bestimmte Reinigungsmittel, Desinfektionsmittel und Spülmittel können ebenfalls zur Behandlung der Haut verwendet werden, wie schon die topischen Zubereitungen. Sie dienen aber vorzugsweise zur Behandlung von Körperhöhlen, Wunden und auch des Mund- und Rachenraumes sowie der Nase.

Die erfindungsgemäßen Wirkstoffkombinationen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z.B. Nagellacke, Nagellackentferner, Nagelbalsame) und dergleichen.

Es ist auch möglich und gegebenenfalls vorteilhaft, die erfindungsgemäßen Wirkstoffkombinationen mit anderen Wirkstoffen zu kombinieren, beispielsweise mit anderen antimikrobiell, antimycotisch bzw. antiviral wirksamen Stoffen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,5 zu wählen.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdikkungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:

- Wasser oder wäßrige Lösungen,
- Öle, wie Triglyceride der Caprin- oder der Caprylsäure, vorzugsweise aber Rizinusöl,
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren,
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die erfindungsgemäßen Zubereitungen können auch Antioxidantien enthalten.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. $\alpha$-Carotin, $\beta$-Carotin,

Lycopin) und deren Derivate, Liponsäure und deren Derivate (z.B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10,0 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwassertoffe (FCKW).

Bevorzugt können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.
Die UVB-Filter können öl-löslich oder wasserlöslich sein. Als öl-lösliche Substanzen sind z.B. zu nennen:

- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;

- Ester der Benzalmalonsäure, vorzugsweise 4-Methoxybenzalmalonsäuredi(2-ethyl0hexyl)ester;
- 2,4,6-Trianilino-(p-carbo-2'-ethyl-1'-hexyloxy)-1,3,5-triazin

Als wasserlösliche Substanzen sind vorteilhaft:

- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxy-benzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Erfindungsgemäße kosmetische und/oder dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nicht-ionische oder amphotere oberflächenaktive Substanz oder Gemische daraus, erfindungsgemäße Wirkstoffe und Hilfsmittel, wie sie üblicherweise dafür verwendet werden.

Beispiele für oberflächen aktive Substanzen, die erfindungsgemäß vorteilhaft verwendet werden können, sind herkömmliche Seifen, z.B. Fettsäuresalze des Natriums, Alkylsulfate, Alkylethersulfate, Alkan- und Alkylbenzolsulfonate, Sulfoacetate , Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate.

Die oberflächenaktive Substanz kann in einer Konzentration zwischen 1 Gew.-% und 50 Gew.-% in dem Shampoonierungsmittel, bzw. der Wasch-, Dusch- oder Badezubereitung, vorliegen.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische davon, sowie erfindungsgemäße Wirkstoffe. Die Menge der verwendeten erfindungsgemäßen Wirkstoffe liegt z.B. zwischen 0,1 und 10 Gew.-%, bevorzugt zwischen 0,1 und 3 Gew.-%.

Kosmetische und dermatologische Zubereitungen zur Behandlung und Pflege der Haare, die die erfindungsgemäßen Wirkstoffe enthalten, können als Emulsionen vorliegen, die vom nicht-ionischen oder anionischen Typ sind. Nicht-ionische Emulsionen enthalten neben Wasser Öle oder Fettalkohole, die beispielsweise polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vom Typ einer Seife und enthalten mindestens eine erfindungsgemäße ethoxylierte oder propoxylierte organische Verbindung mit anionischem oder nicht-ionischem Charakter.

Kosmetische und dermatologische Zubereitungen zur Behandlung und Pflege der Haare können als Gele vorliegen, die neben erfindungsgemäßen Wirkstoffen und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglykolstearat oder -distearat, enthalten. Das Verdickungsmittel ist im Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Wirkstoffe in einem für die Haare bestimmten Mittel 0,01 Gew.-% bis 10 Gew.%, insbesondere 0,5 Gew.-% bis 5 Gew.-%, bezogen auf das Gesamtgewicht der Zubereitungen.

Soweit nicht anders angegeben, beziehen sich Mengenangaben, Prozentangaben und Teile auf das Gewicht, insbesondere auf das Gesamtgewicht der jeweiligen Mischung oder Zubereitung.

Die folgenden Beispiele sollen die Verkörperungen der vorliegenden Erfindungen verdeutlichen. Die Angaben beziehen sich stets auf Gewichts-%, sofern nicht andere Angaben gemacht werden.

<u>**Beispiel 1**</u>

| **W/O-Crème** | |
|---|---|
| Paraffinöl | 10,00 |
| Ozokerit | 4,00 |
| Vaseline | 4,00 |
| pflanzliches Öl | 10,00 |
| Wollwachsalkohol | 2,00 |
| Aluminiumstearat | 0,40 |
| Lecithin | 5,00 |
| Laurylalkohol | 1,00 |
| Parfum, Konservierungsstoffe | q.s. |
| Wasser, VES (voll entsalzt) | ad 100,00 |

<u>**Beispiel 2**</u>

| **W/O-Lotion** | |
|---|---|
| Paraffinöl | 25,00 |
| Siliconöl | 2,00 |

(fortgesetzt)

| W/O-Lotion | |
|---|---|
| Ceresin | 1,50 |
| Wollwachsalkohol | 0,50 |
| Glucosesesquiisostearat | 2,50 |
| Ceramid 3 | 1,00 |
| Myristylalkohol | 1,00 |
| Parfum, Konservierungsstoffe | q.s. |
| Wasser, VES | ad 100,00 |

**Beispiel 3**

| O/W-Lotion | |
|---|---|
| Paraffinöl | 5,00 |
| Isopropylpalmitat | 5,00 |
| Cetylalkohol | 2,00 |
| Bienenwachs | 2,00 |
| Ceteareth-20 | 2,00 |
| PEG-20-Glycerylstearat | 1,50 |
| Glycerin | 3,00 |
| Acyl-Ceramid $C_2$ | 2,00 |
| Ölsäure | 0,5 |
| Parfum, Konservierungsstoffe | q.s. |
| Wasser, VES | ad 100,00 |

**Beispiel 4**

| O/W-Crème | |
|---|---|
| Pflanzliches Öl | 10,00 |
| Cetylalkohol | 2,00 |
| Glycerinmonostearat | 1,50 |
| PEG-30-Glycerylstearat | 2,00 |
| Glycerin | 3,00 |
| Isopropylpalmitat | 5,00 |
| Carbopol 980 (neutralisiert) | 0,30 |
| Sphingoceryl LS | 2,00 |

(fortgesetzt)

| O/W-Crème | |
|---|---|
| GML | 1,0 |
| Parfum, Konservierungsstoffe | q.s. |
| Wasser, VES | ad 100,00 |

**Beispiel 5**

| Salbe | |
|---|---|
| Vaseline | 36,00 |
| Ceresin | 10,00 |
| Zinkoxid | 4,00 |
| Pflanzliches Öl | 20,00 |
| Sphingosomeg AL | 3,50 |
| Wollwachssäure | 0,25 |
| Parfum, Konservierungsstoffe | q.s. |
| Paraffinöl | ad 100,00 |

**Beispiel 6**

| Hautöl | |
|---|---|
| Cetylpalmitat | 3,00 |
| $C_{12-15}$-Alkylbenzoat | 2,00 |
| Polyisobuten | 10,00 |
| Squalan | 2,00 |
| Glucocerebrosid | 1,00 |
| GMCy | 0,50 |
| Parfum, Konservierungsstoffe | q.s. |
| Paraffinöl | ad 100,00 |

**Beispiel 7**

| Badeöl | |
|---|---|
| Paraffinöl | 20,00 |
| PEG-40-hydriertes Rizinusöl | 5,00 |
| Lecithin | 5,00 |
| 1,2-Dihydroxyhexan | 1,0 |
| Parfum, Konservierungsstoffe | q.s. |
| Sojaöl | ad 100,00 |

**Beispiel 8**

| Lippenstift | |
|---|---|
| Ceresin | 8,00 |
| Bienenwachs | 4,00 |
| Carnaubawachs | 2,00 |
| Vaseline | 40,00 |
| Hydriertes Rizinusöl | 4,00 |
| Sphingoceryl Wax LS 2958B | 2,00 |
| DMC | 0,70 |
| Parfum, Konservierungsstoffe | q.s. |
| Paraffinöl | ad 100,00 |

**Beispiel 9**

| Pflegemaske | |
|---|---|
| PEG-50 Lanolin | 0,50 |
| Glycerylstearat | 2,00 |
| Sonnenblumenkernöl | 3,00 |
| Bentonit | 8,00 |
| Kaolin | 35,00 |
| Zinkoxid | 5,00 |
| Ceramid 5 | 1,00 |

(fortgesetzt)

| Pflegemaske | |
|---|---|
| Azelainsäure | 3,0 |
| Parfum, Konservierungsstoffe | q.s. |
| Wasser, VES | ad 100,00 |

**Beispiel 10**

| Liposomenhaltiges Gel | |
|---|---|
| Lecithin | 6,00 |
| Pflanzliches Öl | 12,50 |
| Hydrolysiertes Kollagen | 2,00 |
| Xanthan Gum | 1,40 |
| Butylenglycol | 3,00 |
| Liposomes CLR | 1,00 |
| Palmitoleinsäure | 0,2 |
| Parfum, Konseivierungsstoffe | q.s. |
| Wasser, VES | ad 100,00 |

**Beispiel 11**

| Duschpräparat mit Rückfetter | |
|---|---|
| Cocoamidodiacetat | 10,00 |
| Natriumlaurylsulfat | 25,00 |
| Kalium Cocyl Hydrolysiertes Kollagen | 5,00 |
| Macadamianußöl | 5,00 |
| Natriumchlorid | 0,60 |
| Dermatein GSL | 4,00 |
| Phenoxyethanol | 0,2 |
| Parfüm, Konservierungsstoffe | q.s. |
| Wasser, VES | ad 100,00 |

**Beispiel 12**

| **Seifenstück** | |
|---|---|
| Na-Salz aus Talgfettsäuren | 60,00 |
| Na-Salz aus Kokosöl | 28,00 |
| Natriumchlorid | 0,50 |
| Glycoderm | 5,00 |
| Kokosfettsäure | 5,00 |
| Parfum, Konservierungsstoffe | q.s. |
| Wasser, VES | ad 100,00 |

**Beispiel 13**

| **Syndetseife** | |
|---|---|
| Natriumlaurylsulfat | 30,00 |
| Natriumsulfosuccinat | 10,00 |
| Kaliumcocoyl hydrolysiertes Kollagen | 2,00 |
| Dimethicon Copolyol | 2,00 |
| Paraffin | 2,00 |
| Maisstärke | 10,00 |
| Talcum | 10,00 |
| Glycerin | 3,00 |
| Glycocer | 5,00 |
| Glycerinisolaurat | 1,5 |
| Parfum, Konservierungsstoffe | q.s. |
| Wasser, VES | ad 100,00 |

**Beispiel 14**

| **Haarpflegemittel** | |
|---|---|
| TEA-Cocoyl hydrolysiertes Kollagen | 30,00 |
| Monoethanolaminlaurylsulfat | 25,00 |
| Mandelöl | 2,00 |

(fortgesetzt)

| Haarpflegemittel | |
|---|---|
| Natriumchlorid | 1,00 |
| Glycocer MALA | 2,50 |
| Pelargonsäure | 0,20 |
| Parfum, Konservierungsstoffe | q.s. |
| Wasser, VES | ad 100,00 |

**Beispiel 15**

| Pflegeshampoo | |
|---|---|
| Dinatriumlaurylsulfosuccinat | 6,00 |
| Cocoamidopropylbetain | 10,00 |
| Glycoldistearat | 5,00 |
| Globotriaosylceramid | 0,50 |
| Ölsäuremonoglycerid | 0,40 |
| Parfüm, Konservierungsstoffe | q.s. |
| Wasser, VES | ad 100,00 |

**Beispiel 16**

| Haarkur | |
|---|---|
| Cetylalkohol | 5,00 |
| Caprylic/Capric Triglyceride | 3,00 |
| Petrolatum | 2,00 |
| Sphingosin | 0,50 |
| 2-Butyloctansäure | 0,20 |
| Glyceryllanolat | 3,50 |
| Parfum, Konservierungsstoffe | q.s. |
| Wasser, VES | ad 100,00 |

**Beispiel 17**

| Haarspülung | |
|---|---|
| Cocoamidopropylbetain | 5,00 |
| Cetylalkohol | 2,00 |
| Propylenglycol | 2,00 |
| Citronensäure | 0,30 |
| Sphingosin-1-sulfat | 5,00 |
| $\Delta^9$-Pentadecinsäure | 0,1 |
| Parfum, Konservierungsstoffe | q.s. |
| Wasser, VES | ad 100,00 |

**Beispiel 18**

| Haarfestiger | |
|---|---|
| Polyvinylpyrrolidon/Vinylacetat/Vinylpropionat-Copolymer | 5,00 |
| Ethanol | 45,00 |
| Pseudoceramid | 2,00 |
| Dimethylphenylethylcarbinol | 0,20 |
| Parfum, Konservierungsstoffe | q.s. |
| Wasser, VES | ad 100,00 |

**Beispiel 19**

| Frisiercrème | |
|---|---|
| Vaseline | 4,00 |
| Cetearylalkohol | 4,00 |
| PEG-40-hydriertes Rizinusöl | 2,00 |
| Isopropylpalmitat | 5,00 |
| Citronensäure | 1,00 |
| Lecithin | 2,00 |
| Anisalkohol | 0,05 |
| Parfum, Konservierungsstoffe | q.s. |

(fortgesetzt)

| Frisiercrème | |
|---|---|
| Wasser, VES | ad 100,00 |

**Beispiel 20**

| Rasierschaum | |
|---|---|
| Stearinsäure | 7,00 |
| Natriumlaurylsulfat | 3,00 |
| Stearylalkohol | 1,00 |
| Glycerin | 5,00 |
| Triethanolamin | 3,60 |
| Cerebrosidsulfat | 1,00 |
| Anisaldehyd | 0,05 |
| Parfum, Konservierungsstoffe | q.s. |
| Wasser, VES | ad 100,00 |

**Beispiel 21**

| Fußcrème | |
|---|---|
| Soluan 5 | 2,00 |
| Methylsalicylat | 5,00 |
| Caprylic/Capric Triglyceride | 10,00 |
| Stearinsäure | 5,00 |
| Cetylalkohol | 1,00 |
| Glycerin | 2,00 |
| Dimethicon | 1,00 |
| Carbopol 984 | 0,50 |
| Triethanolamin | 1,50 |
| Phospholipids FPA | 1,50 |
| 2-Butyloctansäure | 0,30 |
| Parfum, Konservierungsstoffe | q.s. |
| Wasser, VES | ad 100,00 |

**Beispiel 22**

| Aerosolspray | |
|---|---|
| Octyldodecanol | 0,50 |
| Sphingosin | 1,50 |
| DMC | 0,70 |
| Parfum, Konservierungsstoffe | q.s. |
| Ethanol | ad 100,00 |

Die durch Zusammenmischung der jeweiligen Bestandteile erhaltene flüssige Phase wird zusammen mit einem Propan-Butan-Gemisch (2:7) im Verhältnis 39:61 in Aerosolbehälter abgefüllt.

**Beispiel 23**

| Pumpspray | |
|---|---|
| PEG-40-Hydriertes Rizinusöl | 2,00 |
| Glycerin | 1,00 |
| Lecithin | 2,00 |
| GMC | 0,70 |
| Parfum, Konservierungsstoffe | q.s. |
| Wasser, VES | ad 100,00 |

**Beispiel 24**

| Roll-on-Gel | |
|---|---|
| 1,3-Butylenglycol | 2,00 |
| PEG-40-Hydriertes Rizinusöl | 2,00 |
| Hydroxyethylcellulose | 0,50 |
| Ceramid 2 | 1,00 |
| TML | 1,00 |
| Parfum, Konservierungsstoffe | q.s. |
| Wasser, VES | ad 100,00 |

**Beispiel 25**

| Roll-on-Emulsion | |
|---|---|
| Tricetearethphosphat | 0,30 |
| Octyldodecanol | 2,00 |
| $C_{12-15}$-Alkylbenzoat | 2,00 |
| $C_{10-30}$-Alkylacrylat | 0,15 |
| Ceramid 4 | 1,00 |
| Glycerinisolaurat | 1,50 |
| Parfum, Konservierungsstoffe | q.s. |
| Wasser, VES | ad 100,00 |

**Beispiel 26**

| Wachsstift | |
|---|---|
| Hydriertes Rizinusöl | 5,00 |
| Bienenwachs | 6,00 |
| Ceresin | 30,00 |
| $C_{12-15}$-Alkylbenzoat | 17,00 |
| Ceramid GSL | 7,50 |
| Wollwachssäure | 2,50 |
| Parfum, Konservierungsstoffe | q.s. |
| Octyldodecanol | ad 100,00 |

**Beispiel 27**

**Kapseln**

Kapseln, welche die nachstehend angegebenen Bestandteile enthalten, werden nach bekannten Arbeitsweisen hergestellt. Diese sind für die Behandlung der vor-stehenden Zwecke in Dosierungsmengen von jeweils einer Kapsel einmal oder mehrmals täglich geeignet:

| | |
|---|---|
| Lecithin | 0,50 g; oder |
| Globotetraosylceramid | 0,50 g |

in Kombination mit

| | |
|---|---|
| Linolensäure | 0,10g; oder |
| GML | 0,10g |

Die Zahlenangaben in den vorstehenden und folgenden Beispielen sind Gew.-%.

Die erfindungsgemäßen Wirkstoffe können besonders vorteilhaft in O/W-bikontinuierlichen und W/O-Mikroemulsionen verwendet werden. Kosmetische und dermatologische Zubereitungen gemäß der Erfindung können besonders vorteilhaft als

a) unverdickte,
b) klassisch, z.B. durch Zusatz von Polyoxameren, Pluronics, Carragenanen oder Pflanzengummen verdickte
c) durch Zusatz von A-B-A-Triblockcopolymeren (z.B. PEG-150-Distearat, Fa. Akto Nobel) oder alpha, omega-bis-polyethoxylierte Silane oder Silikone) verdickte,
d) durch Zusatz von Stempolymeren (z.B. PEC-300-Pentaerythrityl-tetrastearat oder hydrophob modifizierte Tetrakis-polyethoxylierte Silane und Silikone) verdickte,
e) durch Zusatz von A-B-A-B-Multiblock-Copolymeren, Starburst-Polymeren, Dendrimeren und anderen supramolekularen Vernetzern (z.B. Rheodol TWIS 399, Fa. KAO, oder PEG-120-Methylglucose-dioleat) verdickte Öl-in-Wasser- (O/W-), bikontinuierliche oder Wasser-in-Öl- (W/O-) Mikroemulsionen Verwendung finden.

**Beispiel 28**

| | |
|---|---|
| 1,3-Di-(2-ethylhexyl)-cyclohexan | 35,00 |
| Lecithin | 5,00 |
| DML | 2,50 |
| Sorbitanmonolaurat | 10,00 |
| Wasser (+ Zitronensäure bis pH5,5) | ad 100,00 |

**Beispiel 29**

| | |
|---|---|
| 1,3-Di-(2-ethylhexyl)-cyclohexan | 33,00 |
| Lysosulfatide | 5,00 |
| Ölsäuremonoglycerid | 1,50 |
| Sorbitanmonolaurat | 10,00 |
| PEG-150-Distearat | 2,00 |
| Wasser (+Zitronensäure bis pH5,5) | ad 100,00 |

**Beispiel 30**

| | |
|---|---|
| Steareth-15 | 4,80 |
| Glycerin-monostearat | 2,40 |
| Sphingosin | 2,50 |
| DMC | 1,00 |

(fortgesetzt)

| | |
|---|---|
| Cyclomethicon | 3,30 |
| Cetearyloctanoat | 1,70 |
| Wasser | ad 100,00 |

**Beispiel 31**

| | |
|---|---|
| Steareth-15 | 4,80 |
| Glycerin-monostearat | 2,40 |
| Psychosin | 2,50 |
| GMCy | 0,80 |
| Cyclomethicon | 3,30 |
| Cetearyloctanoat | 17,00 |
| PEG-150-Distearat | 2,00 |
| Wasser | ad 100,00 |

**Patentansprüche**

**1.** Zubereitungen, insbesondere kosmetische oder dermatologische, insbesondere topische Zubereitungen mit einem Gehalt an einer Kombination von

(A) einem Wirkstoff oder mehreren Wirkstoffen, ausgewählt aus der Gruppe der antiadhäsiv wirkenden Wirkstoffe (Anti-Adhäsiva),
kombiniert mit
(B) einem Wirkstoff oder mehreren Wirkstoffen, ausgewählt aus der Gruppe der antimikrobiell wirkenden Wirkstoffe oder Wirksysteme (Mikrobizide).

**2.** Kombination von Wirkstoffen (A) und (B) gemäß Anspruch 1.

**3.** Verwendung, insbesondere in Zubereitungen, insbesondere in kosmetischen oder dermatologischen, insbesondere topischen Zubereitungen, einer Kombination von

(A) einem Wirkstoff oder mehreren Wirkstoffen, ausgewählt aus der Gruppe der antiadhäsiv wirkenden Wirkstoffe (Anti-Adhäsiva),
kombiniert mit
(B) einem Wirkstoff oder mehreren Wirkstoffen, ausgewählt aus der Gruppe der antimikrobiell wirkenden Wirkstoffe oder Wirksysteme (Mikrobizide),

zur Bekämpfung von Bakterien, Mykota, Viren, Parasiten und Protozoen und zu der Behandlung der mit dem insbesondere kutanen Auftreten dieser Mikroorganismen verbundenen kosmetischen, dermatologischen und medizinischen Phänomene, z.B. Achselgeruch, Körpergeruch, Fußgeruch, Kopfgeruch, Akne, seborrhoische Dermatitis, mikrobielle Superinfektionen bei atopischem Ekzem, Psoriasis und Immunsuppression, sowie zur Konservierung oder bei Wundinfektionen.

**4.** Zubereitungen, Kombinationen und Verwendung gemäß den Ansprüchen 1-3, dadurch gekennzeichnet, daß die Antiadhäsiva (A) natürliche Ceramide und Sphingosine pflanzlichen und tierischen Ursprungs, pflanzliche und tierische Rohstoffe dieselben enthaltend, sowie synthetisch hergestellte Ceramide und Sphingosine (z.B. Acyl-Ceramide, Questamide oder Pseudoceramide), aber auch durch chemische oder biologische Transformation aus

natürlichen oder

**5.** Zubereitungen gemäß den Ansprüchen 1, 3 und 4, dadurch gekennzeichnet, daß die Zubereitungen topische Zubereitungen sind.

**6.** Verwendung der Kombinationen oder Zubereitungen gemäß den Ansprüchen 1-5 als Desodorantien.

**7.** Zubereitungen gemäß den vorstehenden Ansprüchen, dadurch gekennzeichnet, daß die Menge der Antiadhäsiva (A) in den Zubereitungen, insbesondere topischen Zubereitungen, 0,2 bis 20 Gew.-%, bezogen auf das Gesamtgewicht der Zubereitungen, und die Menge der Mikrobizide (B) in den Zubereitungen, insbesondere topischen Zubereitungen, 0,05 bis 5 Gew.-%, bezogen auf das Gesamtgewicht der Zubereitungen, beträgt.